# EUROPÄISCHE PATENTSCHRIFT

(11) **EP 4 309 700 B1**
(45) Veröffentlichungstag und Bekanntmachung des Hinweises auf die Patenterteilung: **19.02.2025**
(21) Anmeldenummer: 23185018.1
(22) Anmeldetag: 12.07.2023
(51) Int. Cl.: A61M 5/145

(54) **SPRITZENPUMPE**
SYRINGE PUMP
POMPE À SERINGUE

(30) Priorität: 19.07.2022 DE 102022117992
(43) Veröffentlichungstag der Anmeldung: 24.01.2024
(73) Patentinhaber: B. Braun Melsungen AG, 34212 Melsungen (DE)
(72) Erfinder: BARTSCH, Stefan, 36286 Neuenstein (DE)
(74) Vertreter: Winter, Brandl - Partnerschaft mbB

(56) Entgegenhaltungen:
- EP-A1- 0 566 825
- EP-A1- 3 222 307
- DE-U1- 20 200 885
- US-A1- 2004 057 855

## Beschreibung

Die vorliegende Offenbarung betrifft eine Spritzenpumpe gemäß dem Oberbegriff des Patentanspruchs 1, insbesondere eine Spritzenpumpe mit einem Spritzenhalter zum Halten eines Spritzenzylinders einer eingelegten Spritze, einer Antriebseinheit zur axialen Verschiebung einer Kolbenstange der Spritze, und einem quer zur Längsrichtung der Spritze mittels einem Bremsaktuator von einer Freigabeposition in eine Bremsposition und zurück bewegbaren Bremselement, welches in der Bremsposition die Kolbenstange kontaktiert.

### Hintergrund der Offenbarung

Bei Spritzenpumpen der vorstehend genannten Bauart, ergibt sich das Problem, dass die bekannten Bremsaktuatoren eine relativ lange Reaktionszeit aufweisen. Nach der Übermittlung eines Bremssignals an den Bremsaktuator dauert es eben diese Reaktionszeit, bis das Bremselement die Kolbenstange kontaktiert und somit in axialer Richtung fixiert. Die Reaktionszeit wirkt sich somit negativ auf eine Dosiergenauigkeit der Spritzenpumpe aus.

Bei der Inbetriebnahme der Spritzenpumpe fährt die Antriebseinheit auf die Kolbenstange auf. Dabei wird die Kolbenstange mittels des Bremselementes gehalten und eine axiale Verschiebung der Kolbenstange verhindert. Dadurch wird eine ungewollte Abgabe von Flüssigkeit beim Auffahren der Antriebseinheit auf die Kolbenstange bei Inbetriebnahme verhindert.

### Stand der Technik

Aus der EP 1 329 232 B1 ist eine solche Spritzenpumpe bekannt. Der dort offenbarte Bremsaktuator weist einen auf einem Schlitten angeordneten Motor auf. Dieser Motor treibt über ein Schneckenrad ein auf dem Schlitten gelagertes Zahnrad mit Schrägverzahnung an. Das Zahnrad ist mit einer Gewindebohrung versehen, die mit einem Gewinde einer Spindelstange in Eingriff steht. Durch Drehen des Zahnrades wird die Spindelstange, die gegen Mitdrehen gesichert ist, axial verschoben. Damit bildet die Spindelstange einen Linearantrieb, an welchem auch das Bremselement selbst angeordnet ist. Der Bremsaktuator weist somit eine komplexe Mechanik auf, durch welche eine relativ hohe Reaktionszeit zu erwarten ist. Weiterhin können sich Temperaturunterschiede oder Verschleiß negativ auf die Reaktionszeit auswirken, so dass die Reaktionszeit dann nicht nur relativ hoch ist, sondern des Weiteren auch nicht in ihrer genauen Höhe bekannt ist, was sich weiterhin negativ auf die Dosiergenauigkeit auswirkt.

### Kurzbeschreibung der Offenbarung

Der vorliegenden Offenbarung liegt die Aufgabe zugrunde, eine Spritzenpumpe bereitzustellen, welche die Probleme des Stands der Technik reduziert oder beseitigt. Insbesondere soll die Dosiergenauigkeit der Spritzenpumpe verbessert werden.

Diese Aufgabe wird durch eine Spritzenpumpe gemäß Anspruch 1 gelöst. Weitere vorteilhafte Ausführungsformen sind Gegenstand der Unteransprüche.

Genauer wird die Aufgabe gelöst, indem der Bremsaktuator ein Piezoelement zur Bewegung des Bremselementes aufweist oder ist. Ein solches Piezoelement weist eine besonders kurze Reaktionszeit auf, so dass dann insbesondere auch die Dosiergenauigkeit der Spritzenpumpe verbessert ist. Das Bremselement wird nach der Übermittlung eines Bremssignals an das Piezoelement mittels des Piezoelements als der Bremsaktuator schnell in seine Bremsposition bewegt, so dass dann die Kolbenstange in ihrer axialen Position fixiert ist und keine weitere Flüssigkeit mittels der Kolbenstange aus dem Spritzenzylinder gepumpt werden kann.

Das Piezoelement weist in einer bevorzugten Ausführungsform der Spritzenpumpe einen Stapel aus mehreren elektrisch parallel geschalteten Piezoplatten auf.

Durch die Ausbildung des Piezoelementes als ein Stapel mit mehreren elektrisch parallel geschalteten Piezoplatten, ist die Längenänderung des Piezoelementes vergrößerbar, die das Piezoelement ausführen kann, wenn eine elektrische Spannung an das Piezoelement angelegt wird.

Bevorzugt weist das Bremselement ein Messer, eine Klinge oder einen Stift zur axialen Arretierung der Kolbenstange in der Bremsposition des Bremselementes auf.

Mittels des Messers/Klinge oder des Stiftes ist das Risiko eines Durchrutschens der Kolbenstange an dem Bremselement minimiert.

Vorteilhafter Weise ist das Piezoelement zu dessen Steuerung und / oder Regelung mit einer vorzugsweise im Spritzenhalter angeordneten Steuerungs- und / oder Regelungseinrichtung verbunden.

Mittels der Steuerungs- und / oder Regelungseinrichtung wird eine Dosierung der mittels der Spritzenpumpe aus dem Spritzenzylinder gepumpten Flüssigkeit ermöglicht. Dazu ist die Steuerungs- und / oder Regelungseinrichtung vorzugsweise auch mit der Antriebseinheit steuerungstechnisch verbunden. Mittels der Steuerungs- und / oder Regelungseinrichtung ist ein Antriebssignal an die Antriebseinheit und ein Bremssignal an den Bremsaktuator übermittelbar. Weiterhin könnte die Steuerungs- und / oder Regelungseinrichtung mit einer Eingabeeinheit datentechnisch verbindbar sein, so dass mittels einer solchen Eingabeeinheit die mittels der Spritze zu dosierende Menge an Flüssigkeit an die Steuerungs- und / oder Regelungseinrichtung der Spritzenpumpe übermittelbar ist.

Weiter bevorzugt ist es, die Größe der Spritze mit Hilfe des von dem Bremselement auf das Piezoelement übertragenen Drucks, des daraus resultierenden, von dem Piezoelement an die Steuerungs- und / oder Regelungseinrichtung übertragenen Druckmesssignals und durch eine mittels der Steuerungs- und / oder Regelungseinrichtung ausführbaren Software zu bestimmen.

Diese Bestimmung der Größe der Spritze stellt eine Möglichkeit dar, Dosierfehler aufgrund der Annahme einer falschen Spritzengröße zu vermeiden.

In einer weiteren Ausführungsform der Spritzenpumpe ist der Spritzenzylinder auf einer Seite, über einen Umfangsbereich von kleiner als 180° des Spritzenzylinders in radialer Richtung mittels des Spritzenhalters abstützbar. Der Spritzenhalter ist auf einer dem Bremselement gegenüberliegenden Seite des Spritzenzylinders angeordnet und eine Brems-Kolbenstange verbindet den Spritzenhalter und das Bremselement miteinander.

Somit ist sichergestellt, dass die Spritze auch beim Einwirken einer Bremskraft von dem Bremselement auf die Kolbenstange der Spitze sicher in Position gehalten wird. Weiterhin ist die Spitze schnell und sicher in die gewünschte Position zur Spritzenpumpe einsetzbar. Die Brems-Kolbenstange ist starr oder zumindest teilweise beweglich ausgeführt.

Der Bremsaktuator ist vorteilhafterweise auf derselben Seite des Spritzenzylinders wie das Bremselement angeordnet. Der Bremsaktuator kontaktiert vorzugsweise das Bremselement. Somit ist die Reaktionszeit weiter verkürzbar. Alternativ ist der Einsatz eines Getriebes (Kraftübertragungszugs) zwischen dem Bremsaktuator und dem Bremselement denkbar.

Weiter bevorzugt ist der Bremsaktuator mittels eines durch die Brems-Kolbenstange verlaufenden Kabels mit der Steuerungs- und / oder Regelungseinrichtung verbunden.

Mittels des Kabels sind Steuerung- und/oder Regelungssignale übertragbar. Denkbar ist auch eine Energieversorgung des Bremsaktuators über das Kabel. Eine solche Energieversorgung ist z.B. auch mit der Steuerungs- und / oder Regelungseinrichtung durchführbar.

Nach einer weiteren, alternativen Ausführungsform der Spritzenpumpe ist der Bremsaktuator auf derselben Seite des Spritzenzylinders wie der Spritzenhalter angeordnet. Dann ist mittels des Bremsaktuators die Brems-Kolbenstange quer zur Längsrichtung der Spritze bewegbar, um das Bremselement von der Freigabeposition in die Bremsposition und zurück zu bewegen.

Somit ist der dem Bremselement zugehörige Bereich der Spitzenpumpe, welcher auf der dem Spritzenhalter gegenüberliegenden Seite des Spritzenzylinders angeordnet ist besonderes klein ausführbar. Weiterhin kann eine elektrische Montage der Spritzenpumpe vereinfacht werden, da alle elektrisch zu versorgenden Bauteile besonderes nah aneinander anordnenbar sind.

Zusammenfassend ist eine solche Spritzenpumpe zu vielfältigen Anwendungen geeignet, wie z.B. um in flüssiger Form vorliegende Medikamente mittels Spritzen z.B. in die Blutbahn oder andere Körperbereiche von Personen zu dosieren.

### Kurzbeschreibung der Figuren

Im Nachfolgenden werden bevorzugte Ausführungsformen unter Bezugnahme auf die anliegenden Figuren näher dargestellt.
Fig. 1 zeigt in einer schematischen Darstellung ein erstes Ausführungsbeispiel der Spritzenpumpe in einer Seitenansicht.
Fig. 2 zeigt in einer schematischen Darstellung einen Ausschnitt des ersten Ausführungsbeispiels der Spritzenpumpe in einer dreidimensionalen Ansicht.
Fig. 3 zeigt in einer schematischen Darstellung ein zweites Ausführungsbeispiel der Spritzenpumpe in einer Seitenansicht.
Fig. 4 zeigt in einer schematischen Darstellung einen Ausschnitt des zweiten Ausführungsbeispiels der Spritzenpumpe in einer dreidimensionalen Ansicht.

### Beschreibung bevorzugter Ausführungsbeispiele

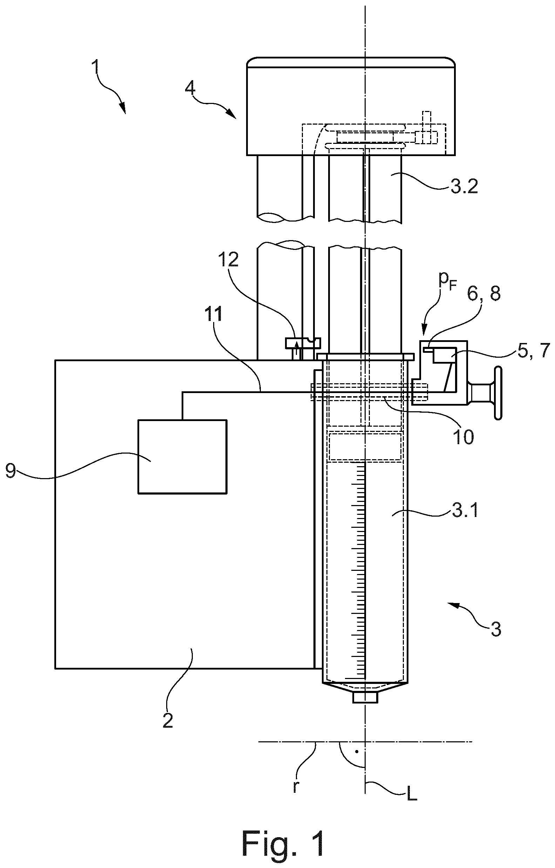

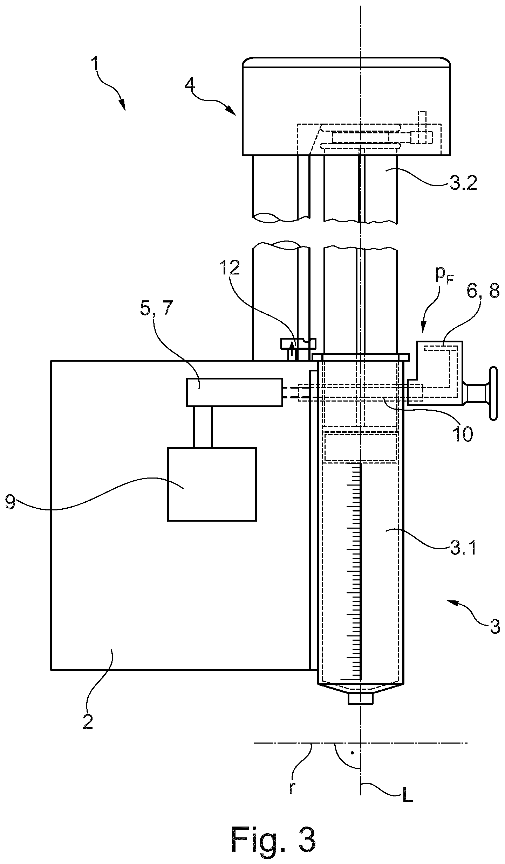

Die Spritzenpumpe 1 hat gemäß Fig.1 und Fig.3 u.a. einen Spritzenhalter 2 zum Halten eines Spritzenzylinders 3.1 einer eingelegten Spritze 3, eine Antriebseinheit 4 zur axialen Verschiebung einer Kolbenstange 3.2 der Spritze 3, und ein quer zur Längsrichtung L der Spritze 3 mittels einem Bremsaktuator 5 von einer Freigabeposition p_{F} in eine Bremsposition p_{B} und zurück bewegbares Bremselement 6, welches in der Bremsposition p_{B} die Kolbenstange 3.2 kontaktiert.

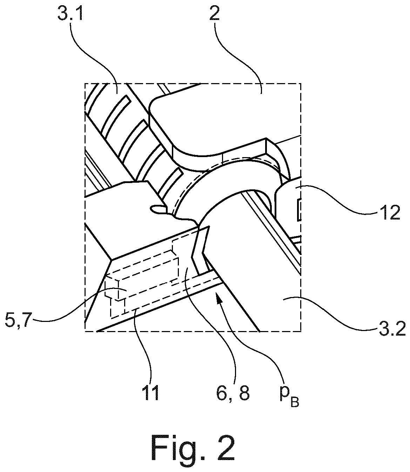

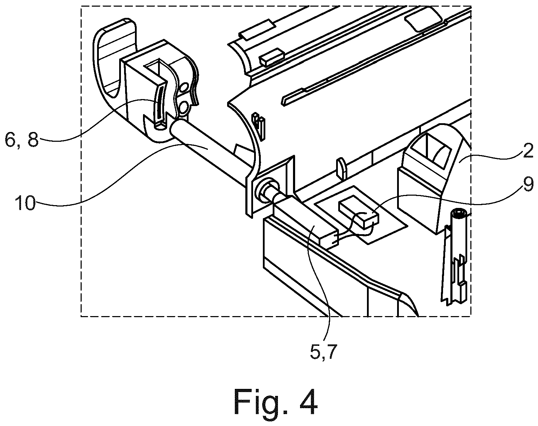

Fig.1 und Fig.3 zeigen das Bremselement 6 in der Freigabeposition p_{F}. In der dreidimensionalen Ansicht des ersten Ausführungsbeispiels der Spritzenpumpe 1 aus Fig. 2 ist das Bremselement 6 in der Bremsposition p_{B} mit Kontakt zur Kolbenstange 3.2 dargestellt. In Fig.4 ist das Innenleben des Spitzenhalters 2 des zweiten Ausführungsbeispiels der Spritzenpumpe 1 gezeigt, wobei die Spritze 3 der Übersichtlichkeit wegen in Fig.4 nicht gezeigt ist. Für gleiche Bauteile werden in allen Figuren gleiche Bezugszeichen verwendet.

Der Bremsaktuator 5 weist ein Piezoelement 7 zur Bewegung des Bremselementes 6 auf.

Um den Bremsaktuator 5 zu betätigen, wird eine Spannung an das Piezoelement 7 angelegt. Dazu sind, wie in den Figuren mittels entsprechender Linien symbolisiert, zwei Adern an das Piezoelement 7 angeschlossen. Je nachdem wie die Spannung gepolt ist, dehnt sich das Piezoelement 7 in seiner axialen Richtung aus oder zieht sich in seiner axialen Richtung zusammen. Mit der axialen Richtung des Piezoelementes 7 ist die Richtung gemeint, in der die Längenänderung des Piezoelementes 7 aufgrund der Kopplung des Piezoelementes 7 mit dem Bremselement 6 zu der Bewegung des Bremselementes 6 führt.

Das Piezoelement 7 weist einen Stapel aus mehreren elektrisch parallel geschalteten Piezoplatten auf, um die erforderliche Längenänderung bei sinnvollen Spannungswerten zu erreichen.

Das Bremselement 6 weist ein Messer 8 oder einen Stift zur axialen Arretierung der Kolbenstange 3.2 in der Bremsposition p_{B} des Bremselementes 6 auf.

Das Piezoelement 7 ist zu dessen Steuerung und / oder Regelung mit einer vorzugsweise im Spritzenhalter 2 angeordneten Steuerungs- und / oder Regelungseinrichtung 9 verbunden. Die Steuerungs- und / oder Regelungseinrichtung 9 ist gemäß Fig.4 auf einer Platine angeordnet, und ist z.B. mittels einer ebenfalls in dem Spritzenhalter 2 anordnenbaren Batterie mit Strom versorgbar. Alternativ könnte die Spritzenpumpe 1 mittels eines Stromkabels mit einer externen Energieversorgung verbunden sein.

Eine Größe der Spritze 3 ist mit Hilfe des von dem Bremselement 6 auf das Piezoelement 7 übertragenen Drucks, des daraus resultierenden von dem Piezoelement 7 an die Steuerungs- und / oder Regelungseinrichtung 9 übertragenen Druckmesssignals und durch eine mittels der Steuerungs- und / oder Regelungseinrichtung 9 ausführbaren Software bestimmbar. Insbesondere ist ein Durchmesser des Spitzenzylinders 3.1 bestimmbar. Vorzugsweise liegt zur Bestimmung der Größe der Spitze 3 in der Steuerungs- und / oder Regelungseinrichtung 9 eine Tabelle vor, in welcher verschiedene Druckmesswerte verschiedenen Spritzengrößen zugeordnet sind. Eine derartige Tabelle wird z.B. vor dem Einsatz der Spitzenpumpe 1 mittels dieser oder einer baugleichen Spritzenpumpe und entsprechenden Spritzen 3 ermittelt und in der Steuerungs- und / oder Regelungseinrichtung 9 hinterlegt. Alternativ könnte auch eine entsprechende funktionale, modellierte Abhängigkeit von Spritzengröße zu Druckmesssignal in der Steuerungs- und / oder Regelungseinrichtung 9 hinterlegt sein.

Der Spritzenzylinder 3.1 ist auf einer Seite, über einen Umfangsbereich von kleiner als 180° des Spritzenzylinders 3.1 in radialer Richtung r mittels des Spritzenhalters 2 abstützbar. Der Spritzenhalter 2 ist auf einer dem Bremselement 6 gegenüberliegenden Seite des Spritzenzylinders 3.1 angeordnet. Eine Brems-Kolbenstange 10 verbindet den Spritzenhalter 2 und das Bremselement 6 miteinander.

Der Bremsaktuator 5 ist gemäß des ersten Ausführungsbeispiels der Spritzenpumpe 1 aus Fig.1 und Fig.2 auf derselben Seite des Spritzenzylinders 3.1 wie das Bremselement 6 angeordnet. Vorzugsweise kontaktiert der Bremsaktuator 5 das Bremselement 6. Z.B. ist das Messer 8 oder der Stift direkt auf dem Piezoelement 7 angeordnet und mit dem Piezoelement 7 verbunden. Die axiale Richtung des Piezoelementes 7 verläuft dann parallel zur Bewegungsrichtung des Bremselementes 6. Vorzugsweise verlaufen die Bewegungsrichtung des Bremselementes 6 und die axiale Richtung der Kolbenstange 3.2 im Wesentlichen senkrecht zueinander.

Der Bremsaktuator 5 ist dann gemäß Fig.1 und Fig.2 mittels eines durch die Brems-Kolbenstange 10 verlaufenden Kabels 11 mit der Steuerungs- und / oder Regelungseinrichtung 9 verbunden.

Das Kabel 11 ist vorzugsweise zweiadrig ausgeführt. Die Polung der an dem Piezoelement 7 anliegenden Spannung ist gemäß Fig.1 und Fig.2 vorzugsweise so gewählt, dass sich das Piezoelement 7 bei anliegender Spannung ausdehnt und somit dann das Bremselement 6 von seiner Freigabeposition p_{F} in die Bremsposition p_{B} bewegt bzw. nachfolgend in der Bremsposition p_{B} gehalten wird. Alternativ ist demgegenüber auch denkbar, dass die Polung so gewählt ist, dass das Bremselement 6 sich in der Bremsposition p_{B} befindet, wenn keine Spannung an das Piezoelement 7 angelegt wird. Beim Anlegen einer Spannung an das Piezoelement 7 wird dann durch axiales Zusammenziehen des Piezoelementes 7 das Bremselement 6 von der Bremsposition p_{B} in die Freigabeposition p_{F} geführt bzw. nachfolgend in der Freigabeposition p_{F} gehalten. Die Polung wird dabei vorzugsweise so gewählt, dass im Betrieb der Spritzenpumpe 1 das Piezoelement 7 über einen längeren Zeitraum nicht mit Strom beaufschlagt werden muss als es mit Strom beaufschlagt werden muss.

Der Bremsaktuator 5 ist gemäß des zweiten Ausführungsbeispiels der Spritzenpumpe aus Fig.3 und Fig.4 auf derselben Seite des Spritzenzylinders 3.1 wie der Spritzenhalter 2 angeordnet. Mittels des Bremsaktuators 5 ist die Brems-Kolbenstange 10 quer zur Längsrichtung L der Spritze 3 bewegbar, um das Bremselement 6 von der Freigabeposition p_{F} in die Bremsposition p_{B} und zurück zu bewegen.

Insbesondere weist die Brems-Kolbenstange 10 dann eine starre holzylindrisch ausgeführte Hülle und einen axial beweglichen, vorzugsweise zylindrisch ausgeführten Kern auf, wobei dann der Kern mit dem Bremsaktuator 5, insbesondere mit dem Piezoelement 7, verbunden ist.

Die Polung der an dem Piezoelement 7 anliegenden Spannung ist gemäß Fig.3 und Fig.4 vorzugsweise so gewählt, dass sich das Piezoelement 7 bei anliegender Spannung zusammenzieht und somit dann das Bremselement 6 von seiner Freigabeposition p_{F} in die Bremsposition p_{B} bewegt bzw. nachfolgend in der Bremsposition p_{B} gehalten wird. Alternativ ist demgegenüber auch denkbar, dass die Polung so gewählt ist, dass das Bremselement 6 sich in der Bremsposition p_{B} befindet, wenn keine Spannung an das Piezoelement 7 angelegt wird. Beim Anlegen einer Spannung an das Piezoelement 7 wird dann durch axiale Ausdehnung des Piezoelementes 7 das Bremselement 6 von der Bremsposition p_{B} in die Freigabeposition p_{F} geführt bzw. nachfolgend in der Freigabeposition p_{F} gehalten. Die Polung wird auch in diesem zweiten Ausführungsbeispiel der Spritzenpumpe 1 vorzugsweise so gewählt, dass im Betrieb der Spritzenpumpe 1 das Piezoelement 7 über einen längeren Zeitraum nicht mit Strom beaufschlagt werden muss als es mit Strom beaufschlagt werden muss.

Die Spritzenpumpe 1 weist vorzugsweise einen Klemmmechanismus 12 auf, mittels welchem der Spritzenzylinder 3.1 nach Einlegen der Spritze 3 in die Spritzenpumpe 1 fixierbar ist. Insbesondere ist mittels des Klemmmechanismus 12 ein am Spritzenzylinder 3.1 ausgebildeter Spritzenflügel mittels des Klemmmechanismus 12 einklemmbar. Um die Spritze 3 aus der Spritzenpumpe 1 zu entnehmen, wird dann zunächst der Klemmmechanismus 12 gelöst.

### Bezugszeichenliste

- 1: Spritzenpumpe
- 2: Spritzenhalter
- 3: Spritze
- 3.1: Spritzenzylinder der Spritze 3
- 3.2: Kolbenstange der Spritze 3
- 4: Antriebseinheit
- 5: Bremsaktuator
- 6: Bremselement
- 7: Piezoelement
- 8: Messer
- 9: Steuerungs- und / oder Regelungseinrichtung
- 10: Brems-Kolbenstange
- 11: Kabel
- 12: Klemmmechanismus

- L: Längsrichtung der Spritze 3
- r: radiale Richtung der Spritze 3
- p_{F}: Freigabeposition des Bremselementes 6
- p_{B}: Bremsposition des Bremselementes 6

## Patentansprüche

1. Spritzenpumpe (1) mit einem Spritzenhalter (2) zum Halten eines Spritzenzylinders (3.1) einer eingelegten Spritze (3), einer Antriebseinheit (4) zur axialen Verschiebung einer Kolbenstange (3.2) der Spritze (3), und einem quer zur Längsrichtung (L) der Spritze (3) mittels einem Bremsaktuator (5) von einer Freigabeposition (p_{F}) in eine Bremsposition (p_{B}) und zurück bewegbaren Bremselement (6), welches in der Bremsposition (p_{B}) die Kolbenstange (3.2) kontaktiert, **dadurch gekennzeichnet, dass** der Bremsaktuator (5) ein Piezoelement (7) zur Bewegung des Bremselementes (6) aufweist.

2. Spritzenpumpe (1) nach Anspruch 1, **dadurch gekennzeichnet, dass** das Piezoelement (7) einen Stapel aus mehreren elektrisch parallel geschalteten Piezoplatten aufweist.

3. Spritzenpumpe (1) nach einem der Ansprüche 1 oder 2, **dadurch gekennzeichnet, dass** das Bremselement (6) ein Messer (8) oder einen Stift zur axialen Arretierung der Kolbenstange (3.2) in der Bremsposition (p_{B}) des Bremselementes (6) aufweist.

4. Spritzenpumpe (1) nach einem der vorstehenden Ansprüche, **dadurch gekennzeichnet, dass** das Piezoelement (7) zu dessen Steuerung und / oder Regelung mit einer vorzugsweise im Spritzenhalter (2) angeordneten Steuerungs- und / oder Regelungseinrichtung (9) verbunden ist.

5. Spritzenpumpe (1) nach Anspruch 4, **dadurch gekennzeichnet, dass** eine Größe der Spritze (3) mit Hilfe des von dem Bremselement (6) auf das Piezoelement (7) übertragenen Drucks, des daraus resultierenden von dem Piezoelement (7) an die Steuerungs- und / oder Regelungseinrichtung (9) übertragenen Druckmesssignals und durch eine mittels der Steuerungs- und / oder Regelungseinrichtung (9) ausführbaren Software bestimmbar ist.

6. Spritzenpumpe (1) nach einem der vorstehenden Ansprüche, **dadurch gekennzeichnet, dass** der Spritzenzylinder (3.1) auf einer Seite, über einen Umfangsbereich von kleiner als 180° des Spritzenzylinders (3.1) in radialer Richtung (r) mittels des Spritzenhalters (2) abstützbar ist, der auf einer dem Bremselement (6) gegenüberliegenden Seite des Spritzenzylinders (3.1) angeordnet ist und eine Brems-Kolbenstange (10) den Spritzenhalter (2) und das Bremselement (6) miteinander verbindet.

7. Spritzenpumpe (1) nach Anspruch 6, **dadurch gekennzeichnet, dass** der Bremsaktuator (5) auf derselben Seite des Spritzenzylinders (3.1) wie das Bremselement (6) angeordnet ist und vorzugsweise das Bremselement (6) kontaktiert.

8. Spritzenpumpe (1) nach Anspruch 7, **dadurch gekennzeichnet, dass** der Bremsaktuator (5) mittels eines durch die Brems-Kolbenstange (10) verlaufenden Kabels (11) mit der Steuerungs- und / oder Regelungseinrichtung (9) verbunden ist.

9. Spritzenpumpe (1) nach Anspruch 6, **dadurch gekennzeichnet, dass** der Bremsaktuator (5) auf derselben Seite des Spritzenzylinders (3.1) wie der Spritzenhalter (2) angeordnet ist, und mittels des Bremsaktuators (5) die Brems-Kolbenstange (10) quer zur Längsrichtung (L) der Spritze (3) bewegbar ist, um das Bremselement (6) von der Freigabeposition (p_{F}) in die Bremsposition (p_{B}) und zurück zu bewegen.

## Claims

1. A syringe pump (1) with a syringe holder (2) for holding a syringe cylinder (3.1) of an inserted syringe (3), a drive unit (4) for axial displacement of a piston rod (3.2) of the syringe (3), and a brake element (6) which can be moved transversely to the longitudinal direction (L) of the syringe (3) via a brake actuator (5) from a release position (p_{F} ) into a brake position (p_{B} ) and back and which contacts the piston rod (3.2) in the brake position (p_{B} ), **characterized in that** the brake actuator (5) has a piezo element (7) for moving the brake element (6).

2. The syringe pump (1) according to claim 1, **characterized in that** the piezo element (7) comprises a stack of several piezo plates electrically connected in parallel.

3. The syringe pump (1) according to one of claims 1 or 2, **characterized in that** the brake element (6) has a knife (8) or a pin for axial locking of the piston rod (3.2) in the brake position (p_{B} ) of the brake element (6).

4. The syringe pump (1) according to one of the preceding claims,
**characterized in that** the piezo element (7) is connected to a control device and/or regulation device (9) preferably arranged in the syringe holder (2) for its control and/or regulation.

5. The syringe pump (1) according to claim 4, **characterized in that** a size of the syringe (3) is determinable by means of the pressure transmitted from the brake element (6) to the piezo element (7), by the resulting pressure measurement signal transmitted from the piezo element (7) to the control device and/or regulation device (9), and by software executable via the control device and/or regulation device (9).

6. The syringe pump (1) according to one of the preceding claims,
**characterized in that** the syringe cylinder (3.1) can be supported on one side over a circumferential region of less than 180° of the syringe cylinder (3.1) in the radial direction (r), via the syringe holder (2), which is arranged on a side of the syringe cylinder (3.1) opposite the brake element (6), and a brake piston rod (10) connects the syringe holder (2) and the brake element (6) to one another.

7. The syringe pump (1) according to claim 6, **characterized in that** the brake actuator (5) is arranged on the same side of the syringe cylinder (3.1) as the brake element (6) and preferably contacts the brake element (6).

8. The syringe pump (1) according to claim 7, **characterized in that** the brake actuator (5) is connected to the control device and/or regulation device (9) via a cable (11) running through the brake piston rod (10).

9. The syringe pump (1) according to claim 6, **characterized in that** the brake actuator (5) is arranged on the same side of the syringe cylinder (3.1) as the syringe holder (2), and the brake piston rod (10) is movable transversely to the longitudinal direction (L) of the syringe (3) via the brake actuator (5) in order to move the brake element (6) from the release position (p_{F} ) to the brake position (p_{B} ) and back.

## Revendications

1. Pompe à seringue (1) avec un support de seringue (2) pour maintenir un cylindre de seringue (3.1) d'une seringue (3) insérée, une unité d'entraînement (4) pour déplacer axialement une tige de piston (3.2) de la seringue (3), et un élément de freinage (6) pouvant être déplacé transversalement à la direction longitudinale (L) de la seringue (3) au moyen d'un actionneur de freinage (5) d'une position de libération (p_{F}) à une position de freinage (p_{B}) et inversement, lequel élément de freinage est en contact avec la tige de piston (3.2) dans la position de freinage (p_{B}), **caractérisé en ce que** l'actionneur de freinage (5) présente un élément piézoélectrique (7) pour déplacer l'élément de freinage (6).

2. Pompe à seringue (1) selon la revendication 1, **caractérisée en ce que** l'élément piézoélectrique (7) présente un empilement de plusieurs plaques piézoélectriques connectées électriquement en parallèle.

3. Pompe à seringue (1) selon l'une quelconque des revendications 1 ou 2, **caractérisée en ce que** l'élément de freinage (6) présente un couteau (8) ou une tige pour bloquer axialement la tige de piston (3.2) dans la position de freinage (p_{B}) de l'élément de freinage (6).

4. Pompe à seringue (1) selon l'une quelconque des revendications précédentes, **caractérisée en ce que** l'élément piézoélectrique (7) est connecté, pour sa commande et/ou sa régulation, à un appareil de commande et/ou de régulation (9) disposé de préférence dans le support de seringue (2).

5. Pompe à seringue (1) selon la revendication 4, **caractérisée en ce qu'**une taille de la seringue (3) peut être déterminée à l'aide de la pression transmise par l'élément de freinage (6) à l'élément piézoélectrique (7), du signal de mesure de pression en résultant transmis par l'élément piézoélectrique (7) à l'appareil de commande et/ou de régulation (9) et par un logiciel exécutable au moyen de l'appareil de commande et/ou de régulation (9).

6. Pompe à seringue (1) selon l'une quelconque des revendications précédentes, **caractérisée en ce que** le cylindre de seringue (3.1) peut être soutenu d'un côté, sur une zone circonférentielle inférieure à 180° du cylindre de seringue (3.1) en direction radiale (r), au moyen du support de seringue (2) qui est disposé sur un côté du cylindre de seringue (3.1) opposé à l'élément de freinage (6), et une tige de piston de freinage (10) connecte le support de seringue (2) et l'élément de freinage (6).

7. Pompe à seringue (1) selon la revendication 6, **caractérisée en ce que** l'actionneur de freinage (5) est disposé du même côté du cylindre de seringue (3.1) que l'élément de freinage (6) et est de préférence en contact avec l'élément de freinage (6).

8. Pompe à seringue (1) selon la revendication 7, **caractérisée en ce que** l'actionneur de freinage (5) est connecté à l'appareil de commande et/ou de régulation (9) au moyen d'un câble (11) s'étendant à travers la tige de piston de freinage (10).

9. Pompe à seringue (1) selon la revendication 6, **caractérisée en ce que** l'actionneur de freinage (5) est disposé du même côté du cylindre de seringue (3.1) que le support de seringue (2), et la tige de piston de freinage (10) peut être déplacée au moyen de l'actionneur de freinage (5) transversalement à la direction longitudinale (L) de la seringue (3) pour déplacer l'élément de freinage (6) de la position de libération (p_{F}) à la position de freinage (p_{B}) et inversement.
